# EUROPEAN PATENT SPECIFICATION

(11) **EP 3 820 415 B1**
(45) Date of publication and mention of the grant of the patent: **05.07.2023**
(21) Application number: 19744865.7
(22) Date of filing: 08.07.2019
(51) Int. Cl.: A61F 5/34

(54) **TOOL FOR STIMULATING A PART OF THE SKIN**
WERKZEUG ZUM STIMULIEREN EINES TEILS DER HAUT
OUTIL DE STIMULATION D'UNE PARTIE DE LA PEAU

(30) Priority: 09.07.2018 BE 201805484
(43) Date of publication of application: 19.05.2021
(73) Proprietor: DMT Wellness Solutions BVBA, 2610 Wilrijk (BE)
(72) Inventor: TIMMERMAN, Bram, 2660 Hoboken (BE); DESMIT, Jan, 4851 ER Ulvenhout (NL); MAC PHERSON, Angus, 2980 Tessenderlo (BE)
(74) Representative: Van hunsel, Lieven M.S.
(86) International application number: PCT/IB2019/055789
(87) International publication number: WO 2020/012322

(56) References cited:
- EP-A1- 3 199 129
- WO-A1-03/030808
- WO-A1-2007/137313
- WO-A1-2011/035820
- GB-A- 1 432 945
- US-A1- 2010 042 026

## Description

The present invention concerns a device for stimulating a part of the skin.

In particular, the invention relates to a device which is designed to act on a surface area of a person's skin in order to produce a subcutaneous effect as well in the near part of the person's body.

The intended effect may be, for example, to increase the blood flow in the relevant subcutaneous area, for example to improve health in general or to increase the effectiveness of a slimming diet.

However, it is not excluded according to the invention to use the device to achieve an additional or alternative effect in the subcutaneous area by stimulating the skin.

In a typical embodiment, such a device according to the invention is designed as a carrying strap that can be placed around a body part and that can be worn, for example, while exercising.

On the other hand, the device is also intended to be effective, even if the wearer does not make any particular physical effort, for example when sitting on a chair, lying on a bed or the like during a break.

Such a device to which the invention relates shall contain at least the following elements:
- stimulation means with a stimulation surface on one side whose dimensions correspond to the dimensions of the relevant surface area of the skin of the person on whom the device must act;
- one or several stimulation elements distributed over the stimulation surface and each formed of a hollow compartment that can be pumped full with a medium to bring it into an expanded state and that can be pumped or sucked out again so as to bring it into a constricted or folded state;
- pumping means for supplying and discharging medium to or from the relevant compartments; and,
- tightening means for tightening the stimulation means against a person's body.

Such devices are already known, but there is room for improvement in these devices.

Such known devices are usually intended to increase the blood flow and stimulate slimming, and the stimulation means are usually alternately filled with medium.

On the spot of the filled stimulation means, the skin of the person is pressed, while in the adjacent skin areas, on the spot of temporarily non-filled stimulation means, the skin is left untouched.

As a result, the person's skin undergoes a kind of massage movement wherein parts of it are alternately pressed and left undisturbed.

The stimulation of the skin with such known devices is rather limited and therefore also their effect on the blood flow and slimming.

From D1 (WO 03/030808 or US2005/070405) for example, a device is known that can be used to stimulate the skin.

The device herein creates zones whereby the skin is exposed to a certain amount of pressure in certain zones, and in adjacent zones the skin is exposed to a certain amount of underpressure.

A major disadvantage of the type of device described, however, is that it is designed as a garment or something similar that has to be applied over the body, the device having to be connected very closely, i.e. hermetically sealed, to the body over large parts of the body.

In particular, this is the case on the entire circumference of the device.

The reason for this is that in the embodiments described in D1, the zones where the skin is exposed to an underpressure can only be created if the edges of the device are hermetically sealed to the body.

In the embodiments of devices as described in D1, there is therefore a high risk of leakages from such an underpressure zone to the surrounding atmosphere, or there is even a high risk that, due to a faulty sealing of the device at the edges, there will simply not be any underpressure zones.

An additional disadvantage of the devices described in D1 is that, due to their close connection to the body, which is necessary for them to function properly, they cannot be designed as a carrying strap or a piece of clothing that can easily be put on and taken off.

Another disadvantage of the devices described in D1 is that many separate compartments are required to achieve a finely tuned skin stimulation, and therefore also unrealistically many connections to pumping equipment for pumping up and emptying the many compartments in this case.

Besides, these additional means such as pumping equipment and connections are so large in D3 that, in practice, they can never be worn by a user, for example while exercising.

D2 (GB1432945) describes a device that, like the devices to which the invention relates, has hollow compartments.

However, such a device described in D2 is intended as a surgical corset that serves as a support between the rib cage and a carrier's pelvis, with the aim of reducing stress on the spine.

Such a device as described in D2 is certainly not intended to act dynamically or alternately on a surface area of a person's skin in order to achieve a subcutaneous effect in the near part of the person's body, as is the case in the present invention.

It is a corset to support a person injured in the back.

Inflatable columns are herein inflated which, in their inflated state, provide additional support for the spine in a permanent and thus a static or almost static manner.

The device described is also designed as a preferably air-permeable double fabric, with inflatable compartments sewn into it.

The air permeability of the fabric is necessary for a patient with back injuries, but such an air-permeable fabric can never be used to obtain skin stimulation, as is obtained with a device according to the present invention.

D3 (EP3199129) describes a similar device that also serves as a support for the back or more specifically for the spine.

The device consists of a support frame to which a compression belt can be attached, typically with Velcro.

This compression belt can be brought in a contracted and an expanded state.

Bringing the belt in the expanded state generates a stretching force on the spine by exerting an external force on the support frame.

It is clear that such a device, as described in D3, cannot be used in any meaningful way to dynamically stimulate the skin in order to obtain subcutaneous, positive effects.

Other devices for stimulating the skin are known from EP2482784 and EP2020979, whereby certain zones are created wherein the skin is stimulated or massaged by alternately applying pressure to it and then releasing the skin.

EP2482784 discloses a device that permits slimming by improving blood flow in the skin, with movement by endurance training devices in the fat-burning pulse, with a main body with a securing means in the form of a sleeve for placing around the abdomen, wherein the main body has at least two chamber systems that can be acted on independently by a fluid, is improved by the fact that at least one and preferably two to four chambers of the chamber systems which come to lie on the right-hand side of the body are arranged in a U-shape with upwardly directed arms, whereas at least one and preferably two to four chambers of the chamber systems which come to lie on the left-hand side of the body are arranged in a U-shape with downwardly directed arms. This additionally stimulates peristalsis. Two chamber systems alternately acted on by a fluid are sufficient in this case. It is expedient if the inlet sites for the fluid into the chambers are located near the transverse part of the U-shaped chambers, such that the fluid adopts a preferred direction of flow.

EP2020979 discloses a slimming device that operates by improving cutaneous circulation during activity in the fat burning pulse range carried out on endurance training equipment. Said device comprises a main body with a fastening element in the form of a sleeve which has at least two chambers that are independent of one another and that can be supplied with a fluid independently of one another. The device can be applied to different body parts and comprises a belt which is designed with at least two separate chambers, at least one pump for producing the alternating pressure load and a fastening sleeve which press the main body onto the human body in order to start the operation of the pressure chamber.

The subcutaneous positive effects of this devices are rather limited.

The present invention therefore aims to provide a solution to the aforementioned problem and/or other problems.

More specifically, the invention aims to provide a skin stimulating device that can stimulate a person's skin and its underlying parts to a greater extent.

In particular, it is an aim of the invention to provide such a device for stimulating the skin in the abdominal region.

The aim here is to be able to design the device as a carrying strap that is easy to put on and take off and that functions in a simple manner and without the need for numerous additional means for its operation that can hardly or not at all be worn by a person.

An additional aim of the invention is therefore to achieve a greater effect on the blood circulation and/or slimming by an increased skin stimulation than is currently the case with the known similar devices.

Another aim of the invention is to improve the health of individuals in general, whereby an appropriate skin stimulation has a positive influence on the individual's health in other ways as well.

To this end, the present invention concerns a device according to the preamble of claim 1, where the device is designed as a carrying strap intended to be placed around a person's abdominal area, where the stimulation means are located in a central section of the carrying strap, where, in addition, a surface section of the stimulation surface is formed of a part of at least one of the stimulation elements and one or more intermediate surface sections of the stimulation surface extend between the contours of this stimulation element, where that stimulation element and the intermediate surface sections are shaped in such a way that, when the stimulation means are applied to a person's skin with the tightening means, and the above-mention stimulation element has been expanded, the person's skin is subjected to a positive pressure at the expanded stimulation element and to an underpressure or negative pressure in one or more intermediate areas, where, in particular, the above-mentioned at least one stimulation element is formed of a hollow compartment having a closed contour that completely encloses one or several intermediate surface sections of the stimulation surface, and whereby, when the above-mentioned stimulation element has been expanded by filling it with medium, sections of the stimulation element adjacent to the skin will exert a pressure on the skin, and other opposing sections of the stimulation element will undergo a movement away from these adjacent sections, wherein edges of at least one zone of one or more of the intermediate surface sections which connect to contours of the stimulation element are also subjected to a movement in this movement away, and wherein this zone of the intermediate surface section concerned forms an airtightly sealed zone since an airtightly sealed, circular strip or edge remains around the zone between the stimulation surface and the skin that is composed of sections of the stimulation element in such a way that, during the aforementioned movement away an underpressure is created between the aforementioned sealed zone and the skin that has a sucking effect on the corresponding part of the skin.

A major advantage of such devices according to the invention is that the skin is not only stimulated or massaged by alternately applying pressure to it and then releasing the skin, but that in one or more intermediate zones between the parts of the skin that are pressed by a stimulation element, the skin is sucked to the stimulation surface by the presence of an underpressure in the area of this zone or these zones.

Consequently, with a device according to the invention, the skin and its underlying parts are subjected to a much greater deformation than is the case with the known devices of this type.

In short, the massage effect obtained with such a device according to the invention is much greater, which contributes to an increase in the blood flow and an accelerated slimming, to a greater extent than is the case with the known devices.

There are further indications known from traditional medicine that applying a vacuum to parts of the skin (the so-called "cupping") could have a positive effect on health.

The aforementioned improvement in blood flow is mentioned here, for example, but toxins could also be removed from the body by applying a vacuum.

In any case, such a method is used in traditional medicine for treating a wide range of complaints ranging from anaemia, arthrosis and depression to the treatment of gynaecological complaints, infertility and impotence, as well as gastrointestinal complaints, migraine and coughing.

A device according to the invention stimulates a person's skin in such a way that this skin is also subjected to a vacuum or underpressure, which opens the way for achieving many other effects that cannot be obtained with the known devices.

Another major advantage of a device according to the invention is that at least one stimulation element is formed of a hollow compartment that has a closed contour and that completely encloses one or more intermediate surface sections of the stimulation surface.

This stimulation element can be inflated very easily because it only forms a single hollow compartment.

In this case, the stimulation element on the abdomen presses a line-shaped zone, the device and the abdomen being hermetically sealed in relation to each other and this zone forming a closed contour.

In short, underpressure created under one or several intermediate surface sections of the stimulation surface are very efficiently and securely sealed off from the surrounding ambient air by a zone that forms a closed contour, so that leakage from the underpressure areas to the environment is virtually excluded, unlike with the known devices.

Another advantage of a device according to the invention is that it is easy to carry out as a carrying strap that is easy to put on and take off, since the operation of the device, in particular the sealing between the device and the skin, is ensured by inflating the aforementioned stimulation element and not by tightening the device against the skin of the wearer when the device is put on.

A device according to the invention offers a major advantage in that the inflation of one of the stimulation elements directly results in a positive pressure on the skin on the spot of said stimulation element, while the skin is also subjected to an underpressure in intermediate zones of the skin, without the need for any additional pumping equipment or suction devices or the like.

It is the combination of the fact that an intermediate surface section of the stimulation surface tries to move away from the skin, which movement is created by inflating one of the stimulation elements, and the fact that an airtight seal is ensured around this intermediate surface section that causes the skin to be subjected to an underpressure.

This intermediate surface section acts as a kind of suction cup on the skin, wherein by inflating a nearby stimulation element, this suction cup will try to move in a direction away from the skin, with the result that the skin is being pulled at.

Depending on the situation, during this movement of the aforementioned intermediate surface section between the skin and the intermediate surface section, a removal may effectively occur to a greater or lesser extent, or the skin may remain completely stuck to the intermediate surface section, as it were.

In any case, the expected result of applying an underpressure to the skin is its deformation towards the outside, whereas when a positive pressure is applied, the skin will deform towards the inside.

In a preferred embodiment of a device according to the invention, the device contains at least one stimulation element with a closed contour which completely encloses one or more intermediate surface sections of the stimulation surface, and in which the aforementioned airtightly sealed, circular edge which remains during the corresponding movement away is composed solely of parts of the stimulation element forming a closed contour.

This embodiment of a device according to the invention is very effective in that the skin is pressed on the spot of the above-mentioned closed-contour stimulation element, at least insofar as the stimulation element concerned has been brought into a filled or expanded state, so that, in this state, a very effective, airtightly sealed seal is obtained between the skin and the stimulation element concerned.

In addition, the extent to which the relevant stimulation element can be pumped up or expanded can be chosen at will when designing the device, for example by determining the maximum inflated thickness of the relevant hollow compartment that makes up the stimulation element.

It is clear that, as the stimulation element is designed with a greater maximum thickness, the greater the above-mentioned movement away will be while the stimulation element is being filled with medium, so that the corresponding intermediate surface sections of the stimulation surface are also included in an increasing movement in a direction that deflects from the skin.

A greater above-mentioned movement of these intermediate surface sections wherein the surrounding contour of the stimulation element simultaneously remains hermetically sealed, is associated with the creation of an increasing underpressure and thus with a greater suction effect on the person's skin.

In this way, the right proportions can be sought for as desired during the design phase in order to achieve the desired suction effect on the skin.

In order to better explain the characteristics of the invention, the following preferred embodiments of a device according to the invention are described by way of example only without being limitative in any way, with reference to the accompanying figures, in which:
figure 1 illustrates a device according to the invention, seen in perspective;
figures 2 and 3 respectively show a top view and a bottom view of the device according to the invention from figure 1, according to arrows F2 and F3 respectively;
figure 4 is a side view according to arrow F4 in figure 2 of the same device according to the invention;
figures 5 to 7 show, to a larger scale, the sections indicated by F5, F6 and F7 respectively in figure 4;
figure 8 shows the device from figure 2 again, with a first stimulation element filled with medium this time, which is represented in grey;
figure 9 shows a cross-section according to the line F9-F9 through the device shown in figure 8, in an idealized form and without any deformation of the skin;
figure 10 shows the part indicated by F10 in figure 8 to a larger scale;
figure 11 shows the device in a similar way as in figure 8, wherein this time another stimulation element is filled with medium, which is represented in grey;
figure 12, as in figure 9, shows a cross section according to line F12-F12 through the device shown in figure 11;
figure 13 shows the part of the device indicated by F13 in figure 9 to a larger scale;
figure 14 shows the same part as in figure 13, but this time it shows the deformation of the skin that occurs under the influence of the forces exerted by the device;
figure 15 shows the part indicated by F15 in figure 13 to a larger scale;
figures 16 to 18, in a similar way as in figures 13 to 15, show the corresponding parts for the situation as illustrated in figure 12; and, figures 19 and 20 are top views of alternative embodiments of a device according to the invention.

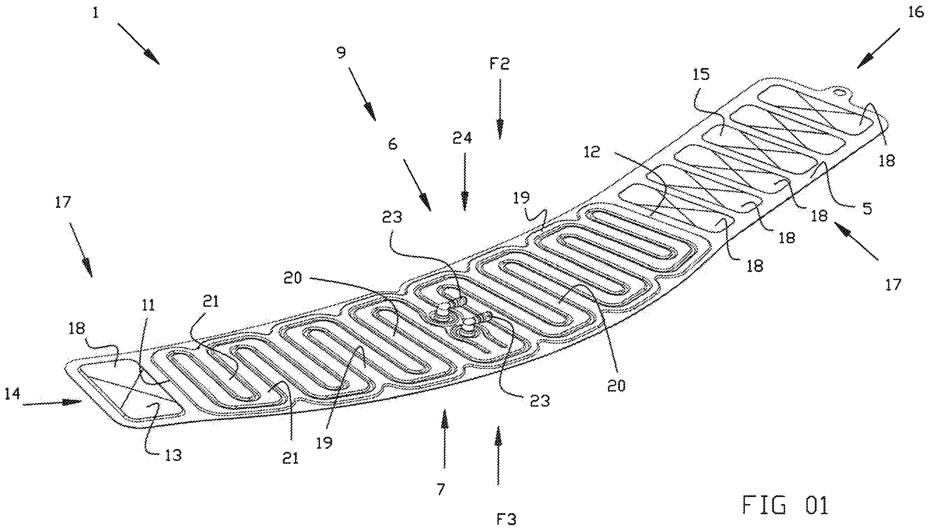

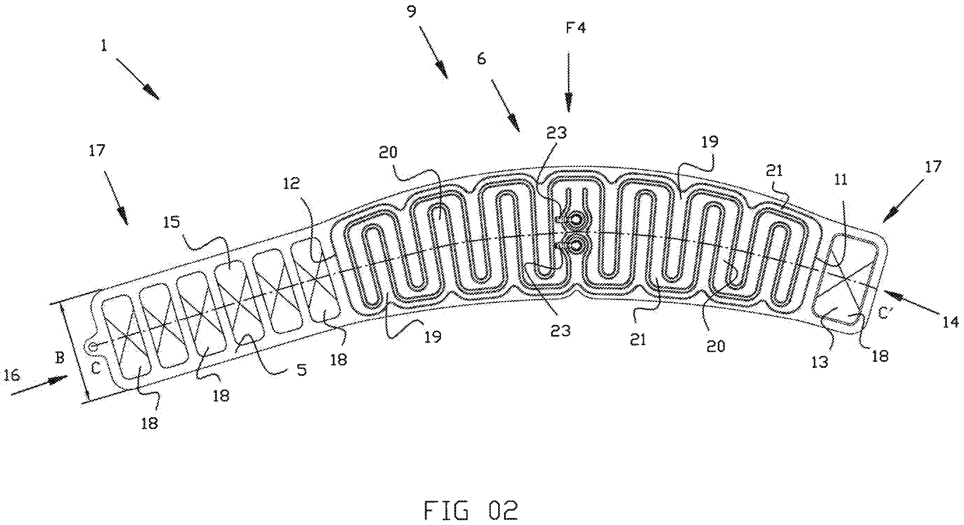

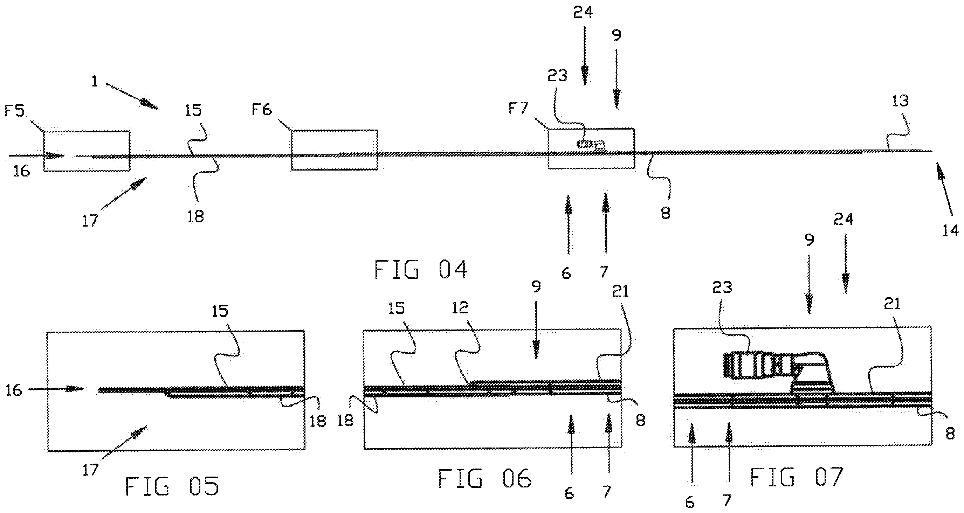

The device 1 according to the invention shown in figures 1 to 7 is intended to act on a surface area 2 of a person's skin 3.

The aim is to also create a subcutaneous effect in the near part 4 of the person's body, wherein this effect can take many forms, as explained in the introduction.

In this case, the device 1 is designed as a carrying strap 5 intended to be placed around the abdominal region of the person.

The device 1 according to the invention is intended to be equipped with stimulation means 6 that have a stimulation surface 8 on one side 7 whose dimensions correspond to the dimensions of the relevant surface area 2 of the skin 3 of the person on whom the device 1 must act.

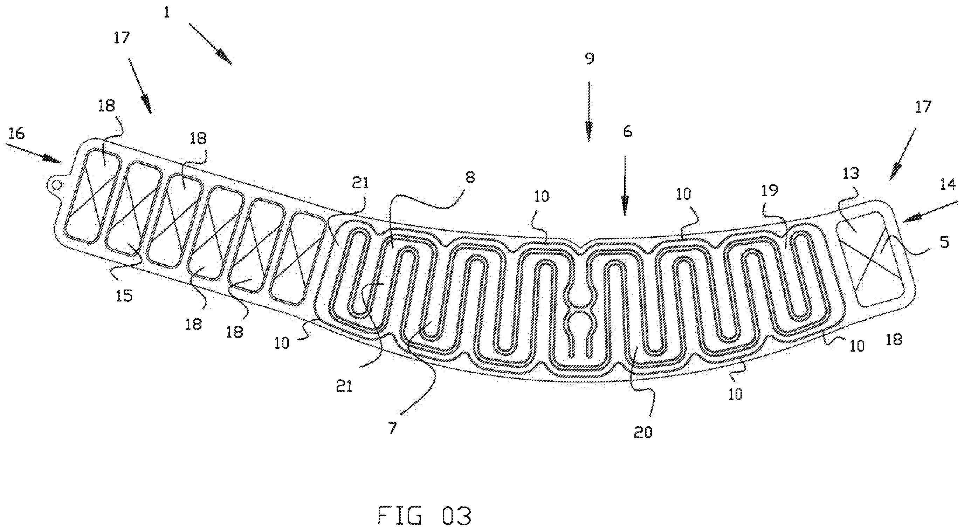

The stimulation means 6 are located in a central section 9 of the carrying strap 5 and the side 7 with the stimulation surface 8 is best represented in this case in the bottom view of figure 3.

This side 7 of the device 1 is the side 7 that should be placed against the abdomen of the person, and the outer contour 10 delineates the stimulation surface 8.

The carrying strap 5 further extends in the length on both sides 11 and 12 of the central section 9, on side 11 on the one hand with a relatively short section 13 forming a first free end 14 of the carrying strap 5, and on side 12 on the other hand with a relatively long section 15 forming a second free end 16 of the carrying strap 5.

The carrying strap 5 has a width B that is constant in the above-mentioned relatively short section 13 and in the above-mentioned relatively long section 15, whereas the width B increases somewhat towards the centre of the central section 9 in which the stimulation means 6 are located.

This width B has typical dimensions which are suitable to be applied on an average abdomen and may vary, for example, between 5 and 20 cm.

The central line CC' of the carrying strap 5 also has a slightly curved course with a section that forms an arc in the central section 9 of the carrying strap 5 or is very close to such an arc.

In the embodiment as represented in the figures, the device 1 is also equipped with tightening means 17 for tightening the stimulation means 6 against a person's body, in this case more specifically against the person's abdomen.

In the example shown, these tightening means 17 are formed of pieces of Velcro 18 provided on the sections 13 and 15 at the free ends 14 and 16 of the carrying strap 5, which can work together to tighten and fasten the carrying strap 5 around the person.

The stimulation means 6 in this case include two stimulation elements 19 and 20 that extend over the stimulation surface 8 and are formed of hollow compartments 21 separated from one another.

These hollow compartments 21 are made of a flexible material and form a kind of hollow bag or tube.

These hollow compartments 21 can be brought in an expanded state by pumping them full with a medium 22, enclosing a maximum volume in the expanded state.

In addition, these hollow compartments 21 may also be placed in a narrowed or folded state by pumping back or sucking out the medium 22, where in this narrowed or folded state the volume enclosed in compartments 21 is zero or nearly zero.

In the embodiment shown in figures 1 to 7, these concave compartments are 21 tubular in shape, extending adjacent to each other and together covering the whole of the stimulation surface 8.

In order to supply medium 22 to the relevant compartments 21 and to discharge it from the relevant compartments 21, a device 1 according to the invention is also equipped with pumping means, which can be carried by the user and which are not shown in any further detail in the figures.

These pumping means are connected to the stimulation elements 19 and 20 by means of tubes, which are not shown either in the figures.

The hollow compartments 21 are each equipped with a T-shaped connector 23 to this end, which is provided on the side 24 opposite the side 7 of the stimulation surface 8, in particular on the side 24 that is turned outwards when carrying the device 1.

In the embodiment of a device 1 according to the invention, as shown in figures 1 to 7, by pumping medium 22 into one of the stimulation elements 19 until it is brought into the expanded state, while the other stimulation element 20 is maintained in the folded state, the person's skin 3 at the expanded stimulation element 19 is subjected to a positive pressure P and, in intermediate zones at the folded stimulation element 20, to an underpressure or negative pressure Q.

More generally, according to the invention, a surface section 25 of the stimulation surface 8 is formed of a section 26 of at least one of the stimulation elements 19, and there are one or several intermediate surface sections 27 of the stimulation surface 8 extending between the contours 28 of this stimulation element 19.

In the case of the figures, these intermediate surface sections 27 are always formed entirely of the other stimulation element 20, which itself can also be put in an expanded or folded state, but this need not necessarily be the case according to the invention. The intermediate surface sections 27 may, for example, consist of several stimulation elements or they may be formed entirely or partly by other elements which are not necessarily hollow and therefore cannot necessarily be expanded or dilated, or folded or narrowed.

It is characteristic of the invention that the above-mentioned stimulation element 19 and the intermediate surface sections 27 are shaped in such a way that, when the stimulation means 6 are applied to a person's skin 3 with the tightening means 17 and the aforementioned stimulation element 19 is brought in the expanded state, the person's skin 3 will be subjected to a positive pressure P at the expanded stimulation element 19, i.e. in zones 29 adjacent to the aforementioned sections 26 of the stimulation element 19, and, in one or several intermediate zones 30, to an underpressure or negative pressure Q.

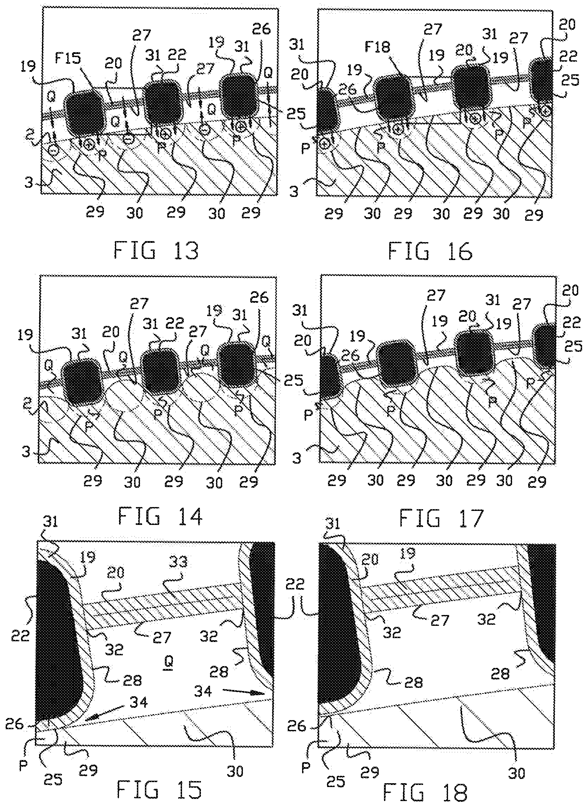

This is illustrated in figures 13 and 15.

In figures 13 and 15, it was just assumed for the sake of convenience that the skin 3 does not deform yet while the stimulation element 19 is being pumped up with medium 22 into the expanded state.

The skin 3 as it may deform under the influence of the forces or pressures P and Q exerted by the stimulation element 19 and the intermediate surface sections 27 is illustrated in figure 14.

It is further noted that when the above-mentioned stimulation element 19 is in an expanded state or is expanded by filling it with medium 22, sections 26 of the stimulation element 19 adjacent to the skin 3 will exert a positive pressure P on the skin 3, while other opposite sections 31 of the stimulation element 19 will undergo a movement away from these adjacent sections 26.

According to the invention, the aim is for the edges 32 of at least one zone 33 of one or several of the intermediate surface sections 27 connecting to contours 28 of the stimulation element 19 to also undergo a movement in this movement away.

In the embodiment of figures 1 to 18, this zone 33 is formed of the entire intermediate surface section 27, or thus the entire stimulation element 20 in the folded state.

This zone 33 of the intermediate surface section 27 concerned forms an airtightly sealed zone 33 as, around the zone 33 between the stimulation surface 8 and the skin 3, an airtightly sealed, circular edge or strip 34 remains.

In the case of the embodiment shown in figures 1 to 18, this edge or strip 34 is composed exclusively of parts of the stimulation element 19, but this is not necessarily the case according to the invention, as will be further explained.

This is possible because the device 1 contains one stimulation element 19 which forms a closed contour completely enclosing the intermediate surface section 27 formed of the second folded stimulation element 20 of the stimulation surface 8.

The result is of course that, during the above-mentioned movement away, an underpressure Q is created between the above-mentioned sealed zone 33 of the intermediate surface section 27 and the skin 3, which has a suction effect on the corresponding area 30 of the skin 3, resulting in the deformation of the skin 3 as shown in figure 14.

As indicated in the introduction, the magnitude of the underpressure Q can be adjusted by varying the maximum thickness or height T of the hollow compartments 21.

Typical sizes of thickness or height T may vary from a few millimetres to a few centimetres.

In this case, the stimulation means 6 contain only a first stimulation element 19 as well as a second stimulation element 20, each formed of a separate tubular compartment 21, with the first stimulation element 19 forming a closed contour around the second stimulation element 20.

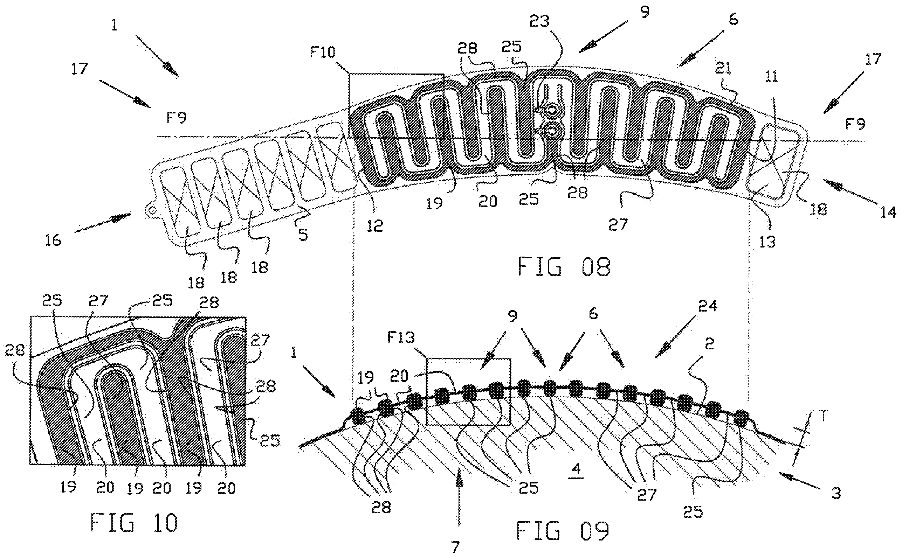

This is clearly illustrated in figure 8 by colouring the stimulation element 19 grey.

In this example, the entire stimulation surface 8 is also formed of sections 26 and 27 of the stimulation elements 19 and 20, which consist of connecting separate compartments 21.

Such an embodiment is obviously very interesting, since it is very easy to manufacture and the means of controlling the two stimulation elements 19 and 20, such as the aforementioned pumping equipment and their means of control, are restricted to a minimum.

Nevertheless, such a structure with only two closely fitting stimulation elements 19 and 20 which together form the entire stimulation surface 8 allows a very intensive stimulation of the abdominal skin 3 and its underlying layers.

This need not necessarily be the case in other embodiments, and a stimulation element may have a closed contour surrounding several other stimulation elements or enclosing one or more other intermediate surface sections 27, which are not necessarily hollow and therefore cannot necessarily be filled with a medium.

In the embodiment shown in figures 1 to 18, the second stimulation element 20 is applied in a wave pattern over the stimulation surface 8, with the first stimulation element 19 completely surrounding this wave pattern, but in other embodiments according to the invention very different patterns can be applied of course.

Naturally, a wave pattern or a branched pattern has the advantage that the stimulation surface is exposed to very different pressures, without having to provide for numerous individual compartments and the associated pumping and control devices.

According to the invention, the pair of stimulation elements 19 and 20 of the stimulation means 6 should be alternately filled with medium 22 and emptied again using the pump media.

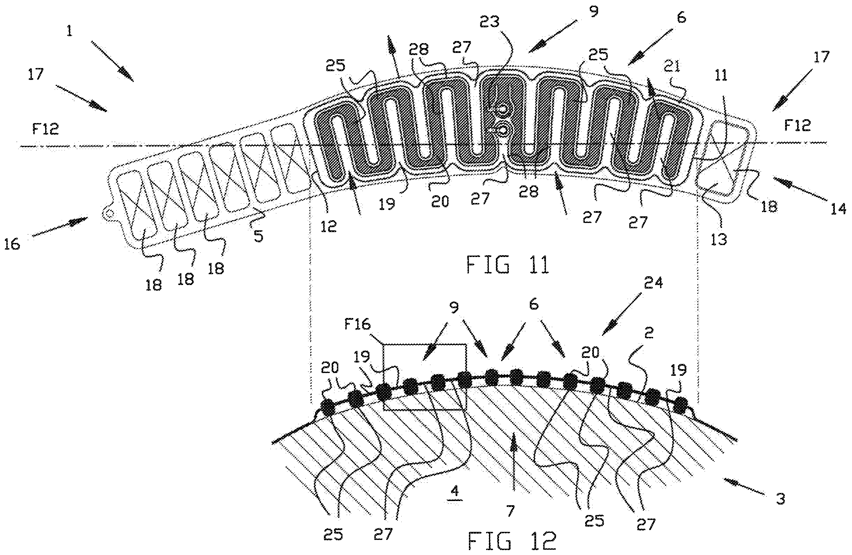

Figures 11 and 12, as well as 16 to 18, illustrate what happens when the other stimulation element 20 is filled with medium 22 and is thus brought in an expanded state.

In this state, the expanded stimulation element 20 can be considered to be a section 25 of the stimulation surface 8, with parts of the folded stimulation element 19 forming intermediate surface sections 27 of this stimulation surface 8 this time.

Sections 31 of the stimulation element 20, when being filled with medium 22, also undergo a movement away as in the previously discussed state in the case where the stimulation element 19 was filled, and the intermediate surface sections 27 of the folded stimulation element 19 are also included in this movement.

As already mentioned, the stimulation element 20 is embodied according to a wave pattern and therefore does not form an enveloping contour around the folded stimulation element 19 in this case, so that in this condition, when the stimulation element 20 is being filled, openings or channels 35 are formed via which ambient air can pass under the intermediate surface sections 27, at least to the extent that the maximum thickness T of the stimulation element 20 allows the formation of such openings or channels 35.

Therefore, in this state of the figures 11 and 12, 16 to 18, no airtightly sealed zone is formed, as in this case no airtightly sealed, circular edge or strip remains while the stimulation element 20 is being filled.

As a result, in this state, no underpressure Q is created below the intermediate surface sections 27, which means that the deformation of the skin 3 in the intermediate zones 30 is this time less pronounced than in the previous state due to the absence of any additional suction force.

This is shown in figures 14 and 17 for comparison.

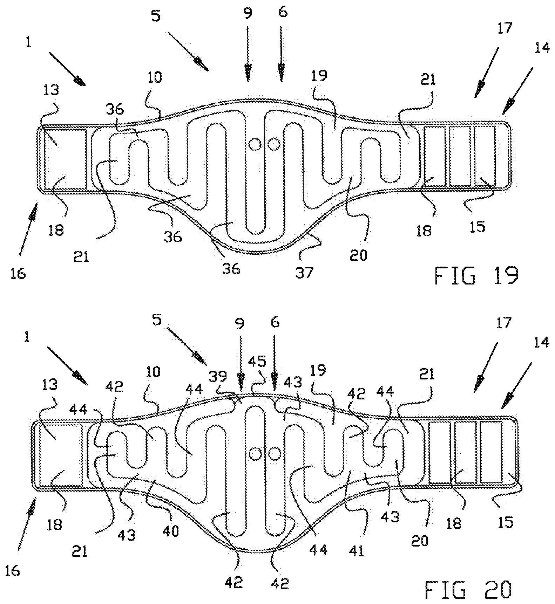

Figure 19 shows another embodiment of a device 1 according to the invention which is very similar to the embodiment of the previous figures.

In this embodiment there is also a first stimulation element 19 that surrounds a second stimulation element 20, wherein the second stimulation element 20 is also applied according to a wave pattern.

However, the wave pattern shows a smaller number of undulations 36 this time, while the width B of the carrying strap 5 increases much more strongly in the central section 9 in this case, with the outer contours 37 of the carrying strap 5 showing a strongly curved course.

Of course, this does not change anything essential to the operation of the device 1 according to the invention.

Figure 20 shows yet another embodiment of a device according to the invention, in which the stimulation surface 8 has been redistributed into two compartments 21 which form a first stimulation element 19 and a second stimulation element 20 and which together make up the entire stimulation surface.

However, the shape of the stimulation element 20 in this case consists of a wave-shaped section 38 with three undulations 39, 40 and 41, i.e. a central undulation 39 and lateral undulations 40 and 41 on both sides thereof.

Centrally in the lateral undulations 40 and 41 is each time provided an additional elongated, finger-shaped protrusion 42 which extends from the belly 43 of the undulation 40 or 41 concerned and which extends parallel to the side arms 44 of the undulation 40 or 41 concerned.

In a similar way, a pair of elongated, finger-shaped protrusions 42 are also provided on the centrally located undulation 39 that extend from the belly 43 thereof, parallel to each other and on the side arms 44 of this undulation 39.

The stimulation element 19 encloses the stimulation element 20 for the most part, but an opening 45 was left at the belly 43 of the centrally located undulation 39 of the stimulation element 20, so that no closed contour is formed around the stimulation element 20.

However, it is clear that in a situation where the partially enveloping stimulation element 19 is filled with medium 22, air supply from the above-mentioned opening 45 parts under the stimulation element 20 that are relatively distant from this opening 45 can be severely obstructed, for example because the skin 3 is sufficiently deformed in some places to prevent the passage.

As a result, in spite of the presence of an opening 45 and the absence of a fully surrounding stimulation element 19, this embodiment may still have an effect as discussed above, whereby under sections of the folded stimulation element 20 an underpressure Q is generated while the stimulation element 19 is being filled with medium 22, thus also achieving the intended effects that were already discussed in the introduction.

It is clear that the examples described above only serve to illustrate what is possible and that many other ways can be used to increase the effect of a device 1 according to the invention on a surface area 2 of the skin 3.

In particular, other techniques can be used to create an underpressure Q on parts of the skin 3.

The invention is by no means limited to the embodiments of a device 1 according to the invention described by way of example and illustrated in the figures; on the contrary, such a device 1 can be realised in other ways while still remaining within the scope of the invention.

## Claims

1. Device (1) intended to act on a surface area (2) of a person's skin (3) in order to produce a subcutaneous effect as well in the near part of the person's body, where the device (1) contains at least the following elements:
- stimulation means (6) with a stimulation surface (8) on one side (7) whose dimensions correspond to the dimensions of the relevant surface area (2) of the skin (3) of the person on whom the device (1) must act;
- one or several stimulation elements (19,20) distributed over the stimulation surface (8) and each formed of a hollow compartment (21) that can be pumped full with a medium (22) to bring it into an expanded state and that can be pumped out or sucked out again so as to bring it into a constricted or folded state;
- pumping means for supplying and discharging medium (22) to or from the relevant compartments (21);
- tightening means (17) for tightening the stimulation means (6) against a person's body,
whereby the device (1) is designed as a carrying strap (5) that is intended to be placed around a person's abdominal region, where the stimulation means (6) are located in a central section (9) of the carrying strap (5), where a surface section (25) of the stimulation surface (8) is formed of a section (26) of at least one of the stimulation elements (19,20) and one or more intermediate surface sections (27) of the stimulation surface (8) extending between the contours (28) of this stimulation element (19,20), where this stimulation element (19,20) and the intermediate surface sections (27) are shaped in such a way that, when the stimulation means are applied to a person's skin with the tightening means, and one or several of the aforementioned stimulation elements (19,20) is brought in the expanded state, the person's skin (3) will be subjected to a positive pressure (P) at the expanded stimulation element (19,20), and to an underpressure or negative pressure (Q) in one or more intermediate zones (30), wherein the aforementioned at least one stimulation element (19,20) is formed of a hollow compartment (21) with a closed contour (10) that entirely encloses the one or more intermediate surface sections (27) of the stimulation surface (8), **characterised in that** when the above-mentioned stimulation element (19,20) has been expanded by filling it with medium (22), sections (26) of the stimulation element (19,20) adjacent to the skin (3) will exert a pressure (P) on the skin (3), and other opposing sections (31) of the stimulation element (19,20) will undergo a movement away from these adjacent sections (26), wherein edges (32) of at least one zone (33) of one or more of the intermediate surface sections (27) which connect to contours (28) of the stimulation element (19,20) are also subjected to a movement in this movement away, and wherein this zone (33) of the intermediate surface section (27) concerned forms an airtightly sealed zone (33) since an airtightly sealed, circular strip or edge (34) remains around the zone (33) between the stimulation surface (8) and the skin (3) that is composed of sections (26) of the stimulation element (19,20) in such a way that, during the aforementioned movement away an underpressure (Q) is created between the aforementioned sealed zone (33) and the skin (3) that has a sucking effect on the corresponding part of the skin (3).

2. Device (1) according to claim 1 , **characterised in that** the aforementioned airtightly sealed, circular edge or strip (34) which remains during the corresponding movement away is composed exclusively of sections (26) of the stimulation element (19) forming a closed contour.

3. Device (1) according to one or several of the preceding claims, **characterised in that**, at the above-mentioned stimulation element (19) having a closed contour (10), the skin (3) is pressed, at least in so far as the relevant stimulation element (19) has been brought in a filled or expanded state, so that in this state a highly effective, airtightly sealed seal is obtained between the skin (3) and the relevant stimulation element (19).

4. Device (1) according to one or several of the preceding claims, **characterised in that** the entire stimulation surface (8) is formed of sections (26,27) of stimulation elements (19,20), consisting of separate, connecting compartments (21) each formed of a hollow compartment (21) which can be pumped full with a medium (22) in order to bring it into an expanded state, and which can be pumped out or sucked out again so as to bring it into a folded state.

5. Device (1) according to one or several of the preceding claims, **characterised in that** the stimulation means (6) contain at least two aforementioned stimulation elements (19,20) which are alternately filled with medium (22) with the aid of the pumping equipment and are emptied again.

6. Device (1) according to one or several of the preceding claims, **characterised in that** the stimulation means (6) contain only a first stimulation element (19) as well as a second stimulation element (20), each formed of a separate tubular compartment (21), with the first stimulation element (19) forming a closed contour around the second stimulation element (20) .

7. Device (1) according to claim 6, **characterised in that** the entire stimulation surface (8) is formed of sections (26) and (27) of the two stimulation elements (19,20), which consist of adjacent separate compartments (21).

8. Device (1) according to claim 6 or 7, **characterised in that** the second stimulation element (19) is applied over the stimulation surface (8) according to a wave pattern.

9. Device (1) according to one or several of the preceding claims, **characterised in that** the carrying strap (5) extends further in the length on both sides (11,12) of the central section (9) with sections (13,15) forming free ends (14,16) of the carrying strap.

10. Device (1) according to one or several of the preceding claims, **characterised in that** the tightening means (17) are made up of Velcro pieces (18) provided on the sections (13,15) at the free ends (14,16) of the carrying strap (5) and which can cooperate to tighten and fasten the carrying strap (5) around the person.

11. Device (1) according to one or several of the preceding claims, **characterised in that** the stimulation means (6) are located in the central section (9) of the carrying strap (5) and wherein the side (7) with the stimulation surface (8) is the side (7) of the device (1) to be placed against the person's abdomen.

12. Device (1) according to one or several of the preceding claims, **characterised in that** the central line (CC') of the carrying strap (5) has a slightly curved course with a section that forms an arc in the central section (9) of the carrying strap (5) or that is very close to such an arc.

13. Device (1) according to one or several of the preceding claims, **characterised in that** the carrying strap (5) has a width (B) that is constant in the sections (13,15) at the free ends (14,16) thereof, whereas the width (B) slightly increases towards the middle of the central section (9) in which the stimulation means (6) are located.

## Patentansprüche

1. Vorrichtung (1), die dazu bestimmt ist, auf eine Oberfläche (2) der Haut (3) einer Person einzuwirken, um auch im nahen Körperteil eine subkutane Wirkung zu erzeugen, wobei die Vorrichtung (1) mindestens die folgenden Elemente enthält:
- Stimulationsmittel (6), die eine Stimulationsfläche (8) auf einer Seite (7) umfassen, deren Abmessungen den Abmessungen der betreffenden Oberfläche (2) der Haut (3) der Person entsprechen, auf die die Vorrichtung (1) wirken muss;
- ein oder mehrere Stimulationselemente (19, 20), die über die Stimulationsfläche (8) verteilt sind und jeweils aus einem mit einem Medium (22) voll pumpbaren Hohlraum (21) gebildet sind, um diesen in einen erweiterten Zustand zu bringen, und der wieder ausgepumpt oder abgesaugt werden kann, um ihn in einen verengten oder gefalteten Zustand zu bringen;
- Pumpmittel zum Zuführen und Austragen des Mediums (22) in die oder aus den betreffenden Räumen (21);
- Festziehungsmittel (17) zum Festziehen der Stimulationsmittel (6) an dem Körper einer Person,
wobei die Vorrichtung (1) als Trageriemen (5) ausgebildet ist, der dazu bestimmt ist, um den Bauchbereich einer Person gelegt zu werden, wobei die Stimulationsmittel (6) sich in einem zentralen Abschnitt (9) des Trageriemens (5) befinden, wobei ein Oberflächenabschnitt (25) der Stimulationsfläche (8) aus einem Abschnitt (26) mindestens eines der Stimulationselemente (19, 20) und einem oder mehreren sich zwischen den Konturen (28) dieses Stimulationselements (19, 20) erstreckenden Zwischenoberflächenabschnitten (27) der Stimulationsfläche (8) gebildet ist, wobei dieses Stimulationselement (19, 20) und die Zwischenflächenabschnitte (27) so geformt sind, dass bei Aufbringen der Stimulationsmittel auf die Haut einer Person mit den Aufspannmitteln und Einbringen eines oder mehrerer der oben genannten Stimulationselemente (19, 20) in den expandierten Zustand, die Haut (3) der Person einem Überdruck (P) an dem expandierten Stimulationselement (19, 20) und einem Unterdruck oder Negativdruck (Q) in einer oder mehreren Zwischenzonen (30) ausgesetzt wird, wobei das zuvor genannte mindestens ein Stimulationselement (19, 20) aus einem Hohlraum (21) gebildet ist, der eine geschlossene Kontur (10) hat, die einen oder mehrere Zwischenflächenabschnitte (27) der Stimulationsfläche (8) vollständig umschließt, **dadurch gekennzeichnet, dass**, wenn das oben genannte Stimulationselement (19, 20) durch das Füllen mit dem Medium (22) erweitert wurde, an die Haut (3) angrenzende Abschnitte (26) des Stimulationselementes (19, 20) einen Druck (P) auf die Haut (3) ausüben werden, und andere gegenüberliegende Abschnitte (31) des Stimulationselements (19, 20) eine Bewegung weg von diesen benachbarten Abschnitten (26) durchmachen werden, wobei Kanten (32) zumindest einer Zone (33) eines oder mehrerer der die Konturen (28) des Stimulationselements (19, 20) verbindenden Zwischenflächenabschnitte (27) auch einer Bewegung weg von dieser Bewegung ausgesetzt sind, und wobei diese Zone (33) des betreffenden Zwischenflächenabschnitts (27) eine luftdicht verschlossene Zone (33) bildet, da ein luftdicht verschlossener, kreisförmiger Streifen oder Rand (34) um den Bereich (33) zwischen der Stimulationsfläche (8) und der Haut (3), der aus Abschnitten (26) des Stimulationselements (19, 20) zusammengesetzt ist, verbleibt, derart, dass im Laufe der oben genannten Wegbewegung ein Unterdruck (Q) zwischen der oben genannten Siegelzone (33) und der Haut (3) entsteht, der auf den entsprechenden Teil der Haut (3) eine Saugwirkung hat.

2. Vorrichtung (1) nach Anspruch 1, **dadurch gekennzeichnet, dass** die oder der oben genannte luftdicht verschlossene, kreisförmige Kante oder Streifen (34), die oder der während der entsprechenden Bewegung wegbleibt, ausschließlich aus Abschnitten (26) des Stimulationselements (19) besteht, die eine geschlossene Kontur bilden.

3. Vorrichtung (1) nach einem oder mehreren der vorhergehenden Ansprüche, **dadurch gekennzeichnet, dass** an dem oben genannten eine geschlossene Kontur (10) umfassenden Stimulationselement (19) die Haut (3) zumindest soweit gedrückt wird, wie das betreffende Stimulationselement (19) in einen gefüllten oder expandierten Zustand gebracht wurde, so dass in diesem Zustand eine hochwirksame, luftdicht verschlossene Abdichtung zwischen der Haut (3) und dem betreffenden Stimulationselement (19) erhalten wird.

4. Vorrichtung (1) nach einem oder mehreren der vorhergehenden Ansprüche, **dadurch gekennzeichnet, dass** die gesamte Stimulationsfläche (8) aus Abschnitten (26,27) von Stimulationselementen (19, 20) gebildet ist, die aus separaten, Verbindungsräumen (21) bestehen, die jeweils aus einem Hohlraum (21) gebildet sind, der mit einem Medium (22) vollgepumpt werden kann, um ihn in einen expandierten Zustand zu bringen, und der wieder ausgepumpt oder abgesaugt werden kann, um ihn in einen gefalteten Zustand zu bringen.

5. Vorrichtung (1) nach einem oder mehreren der vorhergehenden Ansprüche, **dadurch gekennzeichnet, dass** die Stimulationsmittel (6) mindestens zwei oben genannte Stimulationselemente (19, 20) enthalten, die abwechselnd mit Hilfe der Pumpanlage mit dem Medium (22) gefüllt werden und wieder entleert werden.

6. Vorrichtung (1) nach einem oder mehreren der vorhergehenden Ansprüche, **dadurch gekennzeichnet, dass** die Stimulationsmittel (6) nur ein erstes Stimulationselement (19) sowie ein zweites Stimulationselement (20) enthalten, die jeweils aus einem separaten rohrförmigen Raum (21) gebildet sind, wobei das erste Stimulationselement (19) eine geschlossene Kontur um das zweite Stimulationselement (20) bildet.

7. Vorrichtung (1) nach Anspruch 6, **dadurch gekennzeichnet, dass** die gesamte Stimulationsfläche (8) aus Abschnitten (26) und (27) der beiden Stimulationselemente (19, 20) gebildet ist, die aus benachbarten separaten Räumen (21) bestehen.

8. Vorrichtung (1) nach Anspruch 6 oder 7, **dadurch gekennzeichnet, dass** das zweite Stimulationselement (19) gemäß einem Wellenmuster über die Stimulationsfläche (8) aufgebracht ist.

9. Vorrichtung (1) nach einem oder mehreren der vorhergehenden Ansprüche, **dadurch gekennzeichnet, dass** der Trageriemen (5) sich auf beiden Seiten (11, 12) des mittleren Abschnitts (9) weiter in der Länge erstreckt, wobei Abschnitte (13, 15) freie Enden (14, 16) des Trageriemens bilden.

10. Vorrichtung (1) nach einem oder mehreren der vorhergehenden Ansprüche, **dadurch gekennzeichnet, dass** die Aufspannmittel (17) aus an den Abschnitten (13, 15) an den freien Enden (14, 16) des Trageriemens (5) vorgesehenen Klettstücken (18) bestehen, und zusammenwirken können, um den Tragegurt (5) um die Person zu festziehen und zu befestigen.

11. Vorrichtung (1) nach einem oder mehreren der vorhergehenden Ansprüche, **dadurch gekennzeichnet, dass** die Stimulationsmittel (6) sich im mittleren Abschnitt (9) des Trageriemens (5) befinden, und wobei die Seite (7), die die Stimulationsfläche (8) umfasst, die Seite (7) der Vorrichtung (1) ist, die gegen den Bauch der Person gelegt werden muss.

12. Vorrichtung (1) nach einem oder mehreren der vorhergehenden Ansprüche, **dadurch gekennzeichnet, dass** die Mittellinie (CC') des Trageriemens (5) einen leicht gekrümmten Verlauf hat, der einen Abschnitt hat, der im Mittelabschnitt (9) des Trageriemens (5) einen Bogen bildet, oder der einem solchen Bogen sehr nahe ist.

13. Vorrichtung (1) nach einem oder mehreren der vorhergehenden Ansprüche, **dadurch gekennzeichnet, dass** der Trageriemen (5) an seinen freien Enden (14,16) eine Breite (B) hat, die in den Abschnitten (13, 15) konstant ist, während die Breite (B) zur Mitte des Mittelabschnitts leicht zunimmt (9), in dem sich die Stimulationsmittel (6) befinden.

## Revendications

1. Dispositif (1) destiné à agir sur une aire de surface (2) de la peau (3) d'une personne dans le but de produire un effet sous-cutané, de même que dans la partie proche du corps de la personne, dans lequel le dispositif (1) contient au moins les éléments suivants :
- des moyens de stimulation (6) qui comprennent une surface de stimulation (8) sur un côté (7), dont les dimensions correspondent aux dimensions de l'aire de surface pertinente (2) de la peau (3) de la personne, sur laquelle le dispositif (1) doit agir ;
- un ou plusieurs éléments de stimulation (19, 20) qui sont distribués sur la surface de stimulation (8) et qui sont réalisés chacun à partir d'un compartiment creux (21) qui peut être rempli complètement par pompage avec un milieu (22) afin de l'amener dans un état expansé et qui peut être à nouveau vidé par pompage ou par aspiration de manière à l'amener dans un état resserré ou replié ;
- des moyens de pompage destinés à alimenter et à évacuer un milieu (22) en direction ou à partir des compartiments pertinents (21) ;
- des moyens de serrage (17) destinés à serrer les moyens de stimulation (6) contre le corps d'une personne ;
dans lequel le dispositif (1) est conçu sous la forme d'une ceinture de transport (5) qui est destinée à être placée autour de la zone abdominale d'une personne ; dans lequel les moyens de stimulation (6) sont situés dans un tronçon central (9) de la ceinture de transport (5) ; dans lequel un tronçon de surface (25) de la surface de stimulation (8) est réalisé à partir d'un tronçon (26) d'au moins un des éléments de stimulation (19, 20) et d'un ou plusieurs tronçons de surfaces intermédiaires (27) de la surface de stimulation (8) qui s'étendent entre les contours (28) de cet élément de stimulation (19, 20) ; dans lequel cet élément de stimulation (19, 20) et les tronçons de surfaces intermédiaires (27) sont configurés d'une manière telle que, lorsque les moyens de stimulation sont appliqués sur la peau d'une personne avec les moyens de serrage et lorsqu'un ou plusieurs des éléments de stimulation (19, 20) qui ont été mentionnés ci-dessus, est/sont amenés dans l'état expansé, la peau (3) de la personne sera soumise à une pression positive (P) à l'endroit de l'élément de stimulation expansé (19, 20) et à une sous-pression ou une pression négative (Q) dans une ou plusieurs zones intermédiaires (30) ; dans lequel ledit au moins un élément de stimulation (19, 20) qui a été mentionné ci-dessus est réalisé à partir d'un compartiment creux (21) qui comprend un contour fermé (10) qui entoure complètement lesdits un ou plusieurs tronçons de surfaces intermédiaires (27) de la surface de stimulation (8) ; **caractérisé en ce que**, lorsque l'élément de stimulation (19, 20) qui a été mentionné ci-dessus a été soumis à une expansion par le fait de le remplir avec un milieu (22), des tronçons (26) de l'élément de stimulation (19, 20) adjacents à la peau (3) exerceront une pression (P) sur la peau (3) et d'autres tronçons opposés (31) de l'élément de stimulation (19, 20) seront soumis à un mouvement qui s'écarte de ces tronçons adjacents (26) ; dans lequel des bords (32) d'au moins une zone (33) d'un ou de plusieurs des tronçons de surfaces intermédiaires (27) qui relient les contours (28) de l'élément de stimulation (19, 20) sont également soumis à un mouvement qui s'écarte de ce mouvement ; et dans lequel cette zone (33) du tronçon de surface intermédiaire concerné (27) forme une zone (33) scellée de manière étanche à l'air étant donné qu'une bande ou un bord circulaire (34) scellé de manière étanche à l'air subsiste autour de la zone (33) entre la surface de stimulation (8) et la peau (3), qui se compose de tronçons (26) de l'élément de stimulation (19, 20), d'une manière telle qu'au cours du mouvement d'écartement qui a été mentionné ci-dessus, on obtient une sous-pression (Q) entre la zone scellée (33) qui a été mentionnée ci-dessus et la peau (3), qui a un effet d'aspiration sur la partie correspondante de la peau (3).

2. Dispositif (1) selon la revendication 1, **caractérisé en ce que** la bande ou le bord circulaire (34) scellé de manière étanche à l'air qui a été mentionné ci-dessus, qui subsiste au cours du mouvement d'écartement correspondant, se compose exclusivement de tronçons (26) de l'élément de stimulation (19) en formant un contour fermé.

3. Dispositif (1) selon une ou plusieurs des revendications précédentes, **caractérisé en ce que**, à l'élément de stimulation (19) qui a été mentionné ci-dessus, qui possède un contour fermé (10), la peau (3) est comprimée, au moins à un point tel que l'élément de stimulation pertinent (19) a été amené dans un état replié au expansé, d'une manière telle que dans cet état, on obtient un joint étanche à l'air hautement efficace entre la peau (3) et l'élément de stimulation pertinent (19).

4. Dispositif (1) selon une ou plusieurs des revendications précédentes, **caractérisé en ce que** la totalité de la surface de stimulation (9) est réalisée à partir de tronçons (26, 27) d'éléments de stimulation qui sont constitués par des compartiments de liaison séparés (21) réalisés chacun à partir d'un compartiment creux (21) qui peut être rempli complètement par pompage avec un milieu (22) dans le but de l'amener dans un état expansé, et qui peut être à nouveau évacué par pompage ou par aspiration de façon à l'amener dans un état replié.

5. Dispositif (1) selon une ou plusieurs des revendications précédentes, **caractérisé en ce que** les moyens de stimulation (6) contiennent au moins deux éléments de stimulation (19, 20) qui ont été mentionnés ci-dessus qui sont, de manière alternative, remplis avec un milieu (22) à l'aide de l'équipement de pompage et qui sont à nouveau vidés.

6. Dispositif (1) selon une ou plusieurs des revendications précédentes, **caractérisé en ce que** les moyens de stimulation (6) contiennent uniquement un premier élément de stimulation (19) ainsi qu'un deuxième élément de stimulation (20), chacun réalisé à partir d'un compartiment tubulaire séparé (21), le premier élément de stimulation (19) formant un contour fermé autour du deuxième élément de stimulation (20).

7. Dispositif (1) selon la revendication 6, **caractérisé en ce que** la totalité de la surface de stimulation (8) est réalisée à partir de tronçons (26) et (27) des deux éléments de stimulation (19, 20), qui sont constitués par des compartiments séparés adjacents (21).

8. Dispositif (1) selon la revendication 6 ou 7, **caractérisé en ce que** le deuxième élément de stimulation (19) est appliqué sur la surface de stimulation (8) en conformité avec un motif d'ondulation.

9. Dispositif (1) selon une ou plusieurs des revendications précédentes, **caractérisé en ce que** la ceinture de transport (5) s'étend plus loin dans le sens de la longueur des deux côtés (11, 12) du tronçon central (9), avec des tronçons (13, 15) qui forment des extrémités libres (14, 16) de la ceinture de transport.

10. Dispositif (1) selon une ou plusieurs des revendications précédentes, **caractérisé en ce que** les moyens de serrage (17) sont réalisés à partir de bandes velcro (18) qui sont prévues sur les tronçons (13, 15) aux extrémités libres (14, 16) de la ceinture de transport (5) et qui peuvent coopérer pour serrer et fixer la ceinture de transport (5) autour de la personne.

11. Dispositif (1) selon une ou plusieurs des revendications précédentes, **caractérisé en ce que** les moyens de stimulation (6) sont situés dans le tronçon central (9) de la ceinture de transport (5), et dans lequel le côté (7) qui comprend la surface de stimulation (8) représente le côté (7) du dispositif (1) qui doit être placé contre l'abdomen de la personne.

12. Dispositif (1) selon une ou plusieurs des revendications précédentes, **caractérisé en ce que** la ligne centrale (C, C') de la ceinture de transport (5) possède une allure légèrement de forme courbe avec un tronçon qui forme un arc dans le tronçon central (9) de la ceinture de transport (10) ou qui est très proche d'un tel arc.

13. Dispositif (1) selon une ou plusieurs des revendications précédentes, **caractérisé en ce que** la ceinture de transport (5) possède une largeur (B) qui est constante dans les tronçons (13, 15) aux extrémités libres (14, 16) de ces derniers, tandis que la largeur (B) augmente légèrement en direction du milieu du tronçon central (9) dans lequel sont situés les moyens de stimulation (6).
